# EUROPEAN PATENT APPLICATION

(11) **EP 3 943 064 A1**
(43) Date of publication of application: **26.01.2022**
(21) Application number: 21196031.5
(22) Date of filing: 06.02.2009
(51) Int. Cl.: A61K 8/44, A61K 8/21, A61Q 11/00, A61P 1/02, A61K 31/198

(54) **ORAL CARE PRODUCT AND METHODS OF USE AND MANUFACTURE THEREOF**

(30) Priority: 08.02.2008 US 27427
(62) Divisional of application: 09708641.7
(71) Applicant: Colgate-Palmolive Company, New York, NY 10022 (US)
(72) Inventor: ROBINSON, Richard, Belle Mead, 08502 (US); CUMMINS, Diane, Livingston, 07039 (US); SULLIVAN, Richard, Atlantic Highlands, 07716 (US); ELLWOOD, Roger, Flintshire, CH7 5PE (GB)
(74) Representative: Sonnenhauser, Thomas Martin

(57) **Abstract**

This invention relates to methods of treating earh enamel lesions comprising applying an effective amount of a basic amino acid in free or sait form, together w ith fluoride to a patient in need thereof.

## Description

This application claims the benefit of U.S. Ser. No. 61/027,427 filed February 8, 2008, the contents of which are incorporated herein by reference.

### FIELD OF THE INVENTION

This invention relates to methods of oral care using compositions comprising a basic amino acid in free or salt form together with a fluoride source, and to methods of using and of making these compositions.

### BACKGROUND OF THE INVENTION

Arginine and other basic amino acids have been proposed for use in oral care and are believed to have significant benefits in combating cavity formation and tooth sensitivity. Combining these basic amino acids with minerals having oral care benefits, e.g., fluoride and calcium, to form an oral care product having acceptable long term stability, however, has proven challenging. In particular, the basic amino acid may raise the pH and facilitate dissociation of calcium ions that can react with fluoride ions to form an insoluble precipitate. Moreover, the higher pH has the potential to cause irritation. At neutral pH or acidic pH, however, a system utilizing arginine bicarbonate (which the art teaches is preferred) may release carbon dioxide, leading to bloating and bursting of the containers. Moreover, it might be expected that lowering the pH to neutral or acidic conditions would reduce the efficacy of the formulation because the arginine can form an arginine-insoluble calcium complex that has a poorer affinity for the tooth surface, and moreover that lowering the pH would reduce any effect the formulation might have on buffering cariogenic lactic acid in the mouth. Partly because of these unaddressed formulation hurdles and partly because arginine has generally been viewed in the art as a potential alternative to fluoride rather than as a co-active, there has been little motivation to make oral care products comprising both arginine and fluoride. Commercially available arginine-based toothpaste, such as DenClude^{®} and ProClude^{®} containing CaviStat^{®}, for example, contains arginine bicarbonate and calcium carbonate, but no fluoride nor any antimicrobial agent.

Quantitative Light-induced Fluorescence is a visible light fluorescence that can detect early enamel lesions and longitudinally monitor the progression or regression. Normal teeth fluoresce green when illuminated with blue light; demineralized enamel blocks this fluorescence and the area of demineralization can be seen as a dark patch on the tooth, so its size can be quantified and its progress monitored. Areas that have lost mineral have lower fluorescence and appear darker in comparison to a sound tooth surface. Software is used to quantify the fluorescence from a white spot or the area/volume associated with the lesion. Generally, subjects with existing white spot lesions are recruited as panelists. The measurements are performed in vivo with real teeth. The lesion area/volume is measured at the beginning of the clinical. The reduction (improvement) in lesion area/volume is measured at the end of 6 months of product use. The data is often reported as a percent improvement versus baseline.

Electrical Caries Monitoring is a technique used to measure mineral content of the tooth based on electrical resistance. Electrical conductance measurement exploits the fact that the fluid-filled areas of porosity created by demineralization and erosion of the enamel and dentine conduct electricity better than sound areas. As a tooth loses mineral, it becomes less resistive to electrical current due to increased porosity. An increase in the conductance of the patient's teeth therefore may indicate demineralization. Generally, studies are conducted of root surfaces with an existing lesion. The measurements are performed in vivo with real teeth. Changes in electrical resistance before and after 6 month treatments are made. In addition, a classical caries score for root surfaces is made using a tactile probe. The hardness is classified on a three point scale: hard, leathery, or soft. In this type of study, typically the results are reported as electrical resistance (higher number is better) for the ECM measurements and an improvement in hardness of the lesion based on the tactile probe score.

### BRIEF SUMMARY OF THE INVENTION

It is now surprisingly discovered that a basic amino acid such as arginine in combination with fluoride provides unexpected benefits in reducing, repairing or inhibiting early enamel lesions, e.g., as detected by quantitative light-induced fluorescence (QLF) or electronic caries monitor (ECM).

The Compositions of the Invention are thus useful in a method to reduce early lesions of the enamel (as measured by QLF or ECM) relative to a composition lacking effective amounts of fluorine and/or arginine.

Without intending to be bound by a particular theory, it is hypothesized that a significant factor in the beneficial effect of arginine is that arginine and other basic amino acids can be metabolized by certain types of bacteria, e.g., *S. sanguis* which are not cariogenic and which compete with cariogenic bacteria such as *S. mutans,* for position on the teeth and in the oral cavity. The arginolytic bacteria can use arginine and other basic amino acids to produce ammonia, thereby raising the pH of their environment, while cariogenic bacteria metabolize sugar to produce lactic acid, which tends to lower the plaque pH and demineralize the teeth, ultimately leading to cavities, It is believed that regular use of a Composition of the Invention, over time, will lead to a relative increase in the arginolytic bacteria and a relative decrease in the cariogenic bacteria, resulting in a higher plaque pH, in effect immunizing the teeth against cariogenic bacteria and their detrimental effects. It is believed that this pH-raising effect may be mechanistically separate from and complementary to the effect of fluoride in promoting remineralization and strengthening the tooth enamel.

Irrespective of the precise mechanism, however, it is surprisingly found that the combination of fluoride and a basic amino acid, e.g., arginine, in an oral care product produces unexpected benefits beyond and qualitatively different from what can be observed using compositions comprising effective amounts of either compound separately, in promoting remineralization, repairing early enamel lesions before the cavitation process has proceeded into the dentin, and so enhancing oral health. It has moreover been found that this action can be further enhanced by addition of a small particle abrasive, which may act to help fill microfissures in the enamel and microtubules in the dentin.

The invention thus encompasses a method of treating or reducing early enamel caries comprising applying an effective amount of an oral composition comprising a basic amino acid, e.g., arginine, in free or salt form, and an effective amount of fluoride, to the oral cavity of a subject in need thereof.

In one embodiment, the early enamel caries are detected by quantitative light-induced fluorescence (QLF) or electrical caries monitoring (ECM).

In another embodiment, the invention provides a method to improve the QLF or ECM value, e.g., correlating to early enamel lesions, comprising applying an effective amount of a basic amino acid, e.g., arginine, in free or salt form, and an effective amount of fluoride, to the oral cavity of a subject in need thereof.

In another embodiment, the invention provides a method of protecting against cavities comprising measuring the QLF or ECM value for a patient, and improving the QLF or ECM value for such patient to a greater extent than achievable using fluoride without arginine or using arginine without fluoride.

In another embodiment. the invention provides a method to reduce the size of existing early enamel lesions, e.g., as measured by QLF or ECM value, comprising applying an effective amount of a basic amino acid, e.g., arginine, in free or salt form, and an effective amount of fluoride, to the oral cavity of a subject in need thereof.

In another embodiment, the invention provides a method to improve systemic health, e.g., cardiovascular health, through the regular, e.g., daily, application of an oral care product comprising applying an effective amount of a basic amino acid, e.g., arginine, in free or salt form, and an effective amount of fluoride, to the oral cavity of a subject.

### DETAILED DESCRIPTION OF THE INVENTION

The basic amino acid and the fluoride may be administered separately, sequentially or simultaneously. In one embodiment, they are administered as part of a single composition (composition 1.0) comprising an effective amount of a basic amino acid, e.g., arginine, in free or salt form, and an effective amount of fluoride, to the oral cavity of a subject in need thereof, e.g., for example any of the following compositions:
1.0.1. Composition 1.0 wherein the basic amino acid is arginine, lysine, citrullene, ornithine, creatine, histidine, diaminobutanoic acid, diaminoproprionic acid, salts thereof and/or combinations thereof.
1.0.2. Composition 1.0 or 1.0.1 wherein the basic amino acid has the L-configuration.
1.0.3. Any of the preceding compositions is provided in the form of a salt of a di- or tripeptide comprising the basic amino acid.
1.0.4. Any of the preceding compositions wherein the basic amino acid is arginine.
1.0.5. Any of the preceding compositions wherein the basic amino acid is L-arginine.
1.0.6. Any of the preceding compositions wherein the basic amino acid is partially or wholly in salt form.
1.0.7. Composition 1.0.6 wherein the basic amino acid is arginine phosphate.
1.0.8. Composition 1.0.6 wherein the basic amino acid is in the form of arginine hydrochloride.
1.0.9. Composition 1.0.6 wherein the basic amino acid is arginine sulfate.
1.0.10. Composition 1.0.6 wherein the basic amino acid is arginine bicarbonate.
1.0.11. Any of the preceding compositions wherein a salt of the basic amino acid is formed in situ in the formulation by neutralization of the basic amino acid with an acid or a salt of an acid.
1.0.12. Any of the preceding compositions wherein the salt of the basic amino acid is formed by neutralization of the basic amino acid to form a premix prior to combination with the fluoride salt.
1.0.13. Any of the preceding compositions wherein the basic amino acid is present in an amount corresponding to about 0.1 to about 20%, e.g., about 1 wt. % to about 10 wt. % of the total composition weight, the weight of the basic amino acid being calculated as free base form.
1.0.14. Composition 1.0.11 wherein the basic amino acid is present in an amount of about 7.5 wt. % of the total composition weight.
1.0.15. Composition 1.0.11 wherein the basic amino acid is present in an amount of about 5 wt. % of the total composition weight.
1.0.16. Composition 1.0.11 wherein the basic amino acid is present in an amount of about 3.75 wt. % of the total composition weight.
1.0.17. Composition 1.0.11 wherein the basic amino acid is present in an amount of about 1.5 wt. % of the total composition weight.
1.0.18. Any of the preceding compositions wherein the fluoride salt is stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'tris(2-ethanol)-dihydrofluoride), ammonium fluoride, titanium fluoride, hexafluorosulfate, and combinations thereof.
1.0.19. Any of the preceding compositions wherein the fluoride salt is a fluorophosphate.
1.0.20. Any of the preceding composition wherein the fluoride salt is sodium monofluorophosphate.
1.0.21. Any of the preceding compositions where the fluoride salt is sodium fluoride.
1.0.22. Any of the preceding compositions wherein the fluoride salt is present in an amount of about 0.01 wt. % to about 2 wt. % of the total composition weight.
1.0.23. Any of the preceding compositions wherein the fluoride salt provides fluoride ion in an amount of about 0.1 to about 0.2 wt. % of the total composition weight.
1.0.24. Any of the preceding compositions wherein the soluble fluoride salt provides fluoride ion in an amount of from about 50 to about 10,000 ppm.
1.0.25. Any of the preceding compositions which is a mouthwash having about 100 to about 250 ppm available fluoride ion.
1.0.26. Any of the preceding compositions which is a dentifrice having about 750 to about 2000 ppm available fluoride ion.
1.0.27. Any of the preceding compositions wherein the composition comprises about 750 to about 2000 ppm fluoride ion.
1.0.28. Any of the preceding compositions wherein the composition comprises about 1000 to about 1500 ppm fluoride ion.
1.0.29. Any of the preceding compositions wherein the composition comprises about 1450 ppm fluoride ion.
1.0.30. Any of the preceding compositions wherein the pH is about 6 to about 9, e.g., about 6...5 to about 7.4 or about 7.5 to about 9.
1.0.31. Any of the preceding compositions wherein the pH is about 6.5 to about 7.4.
1.0.32. Any of the preceding compositions wherein the pH is about 6.8 and about 7.2.
1.0.33. Any of the preceding compositions wherein the pH is approximately neutral.
1.0.34. Any of the preceding compositions further comprising an abrasive or particulate.
1.0.35. The immediately preceding composition wherein the adhesive or particulate is selected from sodium bicarbonate, calcium phosphate (e.g., dicalcium phosphate dihydrate). calcium sulfate, precipitated calcium carbonate, silica (e.g., hydrated silica), iron oxide, aluminum oxide, perlite, plastic particles, e.g., polyethylene, and combinations thereof.
1.0.36. The immediately preceding composition wherein the abrasive or particulate is selected from a calcium phosphate (e.g., dicalcium phosphate dihydrate), calcium sulfate, precipitated calcium carbonate, silica (e.g., hydrated silica), and combinations thereof.
1.0.37. Any of the preceding compositions comprising an abrasive in an amount of about 15 wt. % to about 70 wt. % of the total composition weight.
1.0.38. Any of the preceding compositions comprising a small particle abrasive fraction of at least about 5% having a d50 of <5 micrometers.
1.0.39. Any of the preceding compositions having an RDA of less than about 150, e.g., about 40 to about 140.
1.0.40. Any of the preceding compositions wherein the anionic surfactant is selected from
   a. water-soluble salts of higher fatty acid monoglyceride monosulfates (e.g., the sodium salt of the monosulfated monoglyceride of hydrogenated coconut oil fatty acids such as sodium N-methyl N-cocoyl taurate, sodium cocomo-glyceride sulfate),
   b. higher alkyl sulfates, e.g., sodium lauryl sulfate,
   c. higher alkyl-ether sulfates, e.g., of formula CH₃(CH₂)ₘCH₂(OCH₂CH₂)ₙOSO₃X, wherein m is 6-16, e.g., 10, n is 1-6, e.g., 2, 3 or 4, and X is Na or K (for example sodium laureth-2 sulfate (CH₃(CH₂)₁₀CH₂(OCH₂CH₂)₂OSO₃Na)),
   d. higher alkyl aryl sulfonates (such as sodium dodecyl benzene sulfonate (sodium lauryl benzene sulfonate)),
   e. higher alkyl sulfoacetates (such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate),
   f. and mixtures thereof.
   By "higher alkyl" is meant, e.g., C₆₋₃₀ alkyl. In particular embodiments, the anionic surfactant is selected from sodium lauryl sulfate and sodium ether lauryl sulfate.
1.0.41. Any of the preceding compositions wherein the anionic surfactant is selected from sodium lauryl sulfate, sodium ether lauryl sulfate. and mixtures thereof.
1.0.42. Any of the preceding compositions wherein the anionic surfactant is present in an amount of from about 0.3% to about 4.5% by weight.
1.0.43. Any of the preceding compositions additionally comprising surfactants selected from cationic, zwitterionic, and nonionic surfactants, and mixtures thereof.
1.0.44. Any of the preceding compositions further comprising at least one humectant.
1.0.45. Any of the preceding compositions further comprising at least one humectant selected from glycerin, sorbitol, xylitol and combinations thereof.
1.0.46. Any of the preceding further compositions comprising xylitol.
1.0.47. Any of the preceding compositions further comprising at least one polymer.
1.0.48. Any of the preceding compositions further comprising at least one polymer selected from polyethylene glycols, polyvinylmethyl ether maleic acid copolymers, polysaccharides (e.g., cellulose derivatives, for example carboxymethyl cellulose, or polysaccharide gums, for example xanthan gum or carrageenan gum), and combinations thereof.
1.0.49. Any of the preceding compositions comprising gum strips or fragments.
1.0.50. Any of the preceding compositions further comprising flavoring, fragrance and/or coloring.
1.0.51. Any of the preceding compositions further comprising water.
1.0.52. Any of the preceding compositions further comprising an antibacterial agent selected from halogenated diphenyl ether (e.g. triclosan), herbal extracts and essential oils (e.g., rosemary extract, tea extract, magnolia extract, thymol, menthol, eucalyptol, geraniol, carvacrol, citral, hinokitol, catechol, methyl salicylate, epigallocatechin gallate, epigallocatechin. gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, metal ions (e.g., zinc salts, for example, zinc citrate, stannous salts, copper salts, iron salts), sanguinarine, propolis and oxygenating agents (e.g., hydrogen peroxide, buffered sodium peroxyborate or peroxycarbonate), phthalic acid and its salts. monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing.
1.0.53. Any of the preceding compositions further comprising an anti-inflammatory compound, e.g., an inhibitor of at least one of host pro-inflammatory factors selected from matrix metalloproteinases (MMP's), cyclooxygenases (COX), PGE₂, interleukin 1 (IL-1), IL-1β converting enzyme (ICE), transforming growth factor β1 (TGF-β1), inducible nitric oxide synthase (iNOS), hyaluronidase, cathepsins, nuclear factor kappa B (NF-κB), and IL-1 Receptor Associated Kinase (IRAK), e.g. selected from aspirin, ketorolac, flurbiprofen, ibuprofen, naproxen, indomethacin, aspirin, ketoprofen, piroxicam, meclofenamic acid, nordihydoguaiaretic acid, and mixtures thereof.
1.0.54. Any of the preceding compositions further comprising an antioxidant, e.g., selected from the group consisting of Co-enzyme Q10, PQQ, Vitamin C, Vitamin E, Vitamin A, anethole-dithiothione, and mixtures thereof.
1.0.55. Any of the preceding compositions wherein the anti-microbial is poorly soluble.
1.0.56. Any of the preceding compositions further comprising triclosan.
1.0.57. Any of the preceding compositions further comprising triclosan and xylitol.
1.0.58. Any of the preceding compositions further comprising triclosan, xylitol, and precipitated calcium carbonate.
1.0.59. Any of the preceding compositions further comprising an antibacterial agent in an amount of about 0.01 to about 5 wt. % of the total composition weight.
1.0.60. Any of the preceding compositions further comprising triclosan in an amount of 0.01 to 1 wt. percent of the total composition weight.
1.0.61. Any of the preceding compositions further comprising triclosan in an amount of about 0.3% of the total composition weight.
1.0.62. Any of the preceding compositions further comprising a whitening agent.
1.0.63. Any of the preceding compositions further comprising a whitening agent selected from a whitening active selected from the group consisting of peroxides, metal chlorites, perborates, percarbonates, peroxyacids, hypochlorites, and combinations thereof
1.0.64. Any of the preceding compositions further comprising hydrogen peroxide or a hydrogen peroxide source, e.g., urea peroxide or a peroxide salt or complex (e.g., such as peroxyphosphate, peroxycarbonate, perborate, peroxysilicate, or persulphate salts; for example calcium peroxyphosphate, sodium perborate, sodium carbonate peroxide, sodium peroxyphosphate, and potassium persulfate).
1.0.65. Any of the preceding compositions further comprising an agent that interferes with or prevents bacterial attachment, e.g., solbrol or chitosan.
1.0.66. Any of the preceding compositions further comprising a source of calcium and phosphate selected from (i) calcium-glass complexes, e.g., calcium sodium phosphosilicates, and (ii) calcium-protein complexes, e.g., casein phosphopeptide-amorphous calcium phosphate.
1.0.67. Any of the preceding compositions further comprising a soluble calcium salt, e.g., selected from calcium sulfate, calcium chloride, calcium nitrate, calcium acetate, calcium lactate, and combinations thereof.
1.0.68. Any of the preceding compositions further comprising a physiologically acceptable potassium salt, e.g., potassium nitrate or potassium chloride, in an amount effective to reduce dentinal sensitivity.
1.0.69. Any of the preceding compositions further comprising from about 0.1% to about 7.5% of a physiologically acceptable potassium salt, e.g., potassium nitrate and/or potassium chloride.
1.0.70. Any of the preceding compositions which is a toothpaste comprising an arginine salt e.g., arginine hydrochloride, arginine phosphate or arginine bicarbonate; triclosan; an anionic surfactant, e.g., sodium lauryl sulfate; and a soluble fluoride salt, e.g., sodium monofluorophosphate or sodium fluoride.
1.0.71. Any of the preceding compositions effective upon application to the oral cavity, e.g., with brushing, to (i) reduce or inhibit formation of dental caries, (ii) reduce, repair or inhibit pre-carious lesions of the enamel, e.g., as detected by quantitative light-induced fluorescence (QLF) or electrical caries measurement (ECM), (iii) reduce or inhibit demineralization and promote remineralization of the teeth, (iv) reduce hypersensitivity of the teeth, (v) reduce or inhibit gingivitis, (vi) promote healing of sores or cuts in the mouth, (vii) reduce levels of acid producing bacteria, (viii) to increase relative levels of arginolytic bacteria, (ix) inhibit microbial biofilm formation in the oral cavity, (x) raise and/or maintain plaque pH at levels of at least pH 5.5 following sugar challenge, (xi) reduce plaque accumulation, (xii) treat, relieve or reduce dry mouth, (xiii) clean the teeth and oral cavity (xiv) reduce erosion, (xv) whiten teeth, (xvi) immunize the teeth against cariogenic bacteria; and/or (xvii) promote systemic health, including cardiovascular health, e.g., by reducing potential for systemic infection via the oral tissues.
1.0.72. A composition obtained or obtainable by combining the ingredients as set forth in any of the preceding compositions.
1.0.73. Any of the preceding compositions in a form selected from mouthrinse, toothpaste, tooth gel, tooth powder, non-abrasive gel, mousse, foam, mouth spray, lozenge, oral tablet, dental implement, and pet care product.
1.0.74. Any of the preceding compositions wherein the composition is toothpaste.
1.0.75. Any of the preceding compositions wherein the composition is a toothpaste optionally further comprising one or more of one or more of water, abrasives, surfactants, foaming agents, vitamins, polymers, enzymes, humectants, thickeners, antimicrobial agents, preservatives, flavorings, colorings and/or combinations thereof.
1.0.76. Any of the preceding compositions 1.0 - 1.0.73 wherein the composition is a mouthwash.
1.0.77. Any of the preceding compositions further comprising a breath freshener, fragrance or flavoring.
1.0.78. Any of the preceding compositions further comprising an anti-calculus agent.
1.0.79. Any of the preceding compositions further comprising an anti-calculus agent which is a polyphosphate, e.g., pyrophosphate, tripolyphosphate, or hexametaphosphate, e.g., in sodium salt form.
1.0.80. Any of the preceding compositions further comprising an effective amount of a salt of a basic amino acid; an effective amount of a soluble fluoride salt; an anionic surfactant, e.g., sodium lauryl sulfate; an anionic polymer, e.g., a copolymer of methyl vinyl ether and maleic anhydride; and an antibacterial agent, e.g., triclosan.
1.0.81. Any of the preceding compositions further comprising an effective amount of a salt of a basic amino acid; an antibacterial agent, e.g., triclosan; an effective amount of a soluble fluoride salt; and small particle abrasive, such that the composition has an RDA of < 160, e.g., about 40 to about 140, e.g., comprising at least about 5%, e.g., at least about 20% of an abrasive having a d50 < 5 micrometers, e.g., silica having a d50 of about 3 to about 4 micrometers. The present invention further provides (i) a combined preparation comprising a basic amino acid, in free or salt form, and a fluoride, for simultaneous, sequential or separate administration to the oral cavity of a subject for treating, reducing or inhibiting early enamel lesions; and (ii) a combined preparation comprising a basic amino acid, in free or salt form, and a fluoride, for simultaneous, sequential or separate administration to the oral cavity of a subject for improving the QLF or ECM value correlating to early enamel lesions. The combined preparation for such is for example a composition is described above or
1.0.82. A combined preparation comprising a basic amino acid, in free or salt form, and a fluoride, for simultaneous, sequential or separate administration to the oral cavity of a subject for treating, reducing or inhibiting early enamel lesions.
1.0.83. A combined preparation according to any foregoing embodiment wherein the basic amino acid is present in an amount of from 0.1 to 20 wt% of the total combined preparation weight.
1.0.84. A combined preparation according to any foregoing embodiment wherein the basic amino acid is present in an amount of from 1 to 10 wt% of the total combined preparation weight.
1.0.85. A combined preparation according to any foregoing embodiment wherein the basic amino acid comprises arginine.
1.0.86. A combined preparation according any foregoing embodiment wherein the arginine is present as a salt of an inorganic oxoacid.
1.0.87. A combined preparation according the foregoing embodiment wherein the inorganic oxoacid is phosphoric acid.
1.0.88. A combined preparation according to any foregoing embodiment wherein the fluoride is present as a soluble fluoride salt in an amount of from 0.01 to 2 wt% of the total combined preparation weight.
1.0.89. A combined preparation according to any foregoing embodiment wherein the fluoride provides a source of fluoride ions in an amount to provide 50 to 25,000 ppm by weight of fluoride ions in the total combined preparation weight.
1.0.90. A combined preparation according to any foregoing embodiment wherein the soluble fluoride salt or source of fluoride ions is selected from sodium fluoride, sodium monofluoropohosphate, and mixtures thereof.
1.0.91. A combined preparation according to any foregoing embodiment further comprising a calcium salt of an inorganic acid, wherein the calcium salt is present in an amount of from 10 to 60wt% of the total combined preparation weight.
1.0.92. A combined preparation according to the foregoing embodiment wherein the calcium salt is a salt of an inorganic oxoacid.
1.0.93. A combined preparation according to the foregoing embodiment wherein the calcium salt comprises calcium phosphate.
1.0.94. A combined preparation comprising a basic amino acid, in free or salt form, and a fluoride, for simultaneous, sequential or separate administration to the oral cavity of a subject for improving the QLF or ECM value correlating to early enamel lesions.
1.0.95. A combined preparation according to any foregoing embodiment wherein the basic amino acid is present in an amount of from 0.1 to 20 wt% of the total combined preparation weight.
1.0.96. A combined preparation according to any foregoing embodiment wherein the basic amino acid is present in an amount of from 1 to 10 wt% of the total combined preparation weight.
1.0.97. A combined preparation according to any foregoing embodiment wherein the basic amino acid comprises arginine.
1.0.98. A combined preparation according any foregoing embodiment wherein the arginine is present as a salt of an inorganic oxoacid.
1.0.99. A combined preparation according the foregoing embodiment wherein the inorganic oxoacid is phosphoric acid.
1.0.100. A combined preparation according to any foregoing embodiment wherein the fluoride is present as a soluble fluoride salt in an amount of from 0.01 to 2 wt% of the total combined preparation weight.
1.0.101. A combined preparation according to any foregoing embodiment wherein the fluoride provides a source of fluoride ions in an amount to provide 50 to 25,000 ppm by weight of fluoride ions in the total combined preparation weight.
1.0.102. A combined preparation according to any foregoing embodiment wherein the soluble fluoride salt or source of fluoride ions is selected from sodium fluoride, sodium monofluoropohosphate, and mixtures thereof.
1.0.103. A combined preparation according to any foregoing embodiment further comprising a calcium salt of an inorganic acid, wherein the calcium salt is present in an amount of from 10 to 60wt% of the total combined preparation weight.
1.0.104. A combined preparation according to the foregoing embodiment wherein the calcium salt is a salt of an inorganic oxoacid.
1.0.105. A combined preparation according to the foregoing embodiment wherein the calcium salt comprises calcium phosphate.
   The present invention thus provides a method (Method 1) of treating or reducing early enamel caries comprising applying an effective amount of an oral composition or combined preparations comprising a basic amino acid, in free or salt form, and an effective amount of fluoride, to the oral cavity, e.g. wherein the basic amino acid is arginine, e.g., wherein the composition or combined preparations is according to any of the foregoing 1.0.1-105.
1.0.106. The method wherein the early enamel caries are detected by quantitative light-induced fluorescence (QLF) or electrical caries monitoring (ECM).
1.0.107. A method to improve the QLF or ECM value, correlating to early enamel lesions, comprising applying an effective amount of a basic amino acid a basic amino acid, arginine, in free or salt form, and an effective amount of fluoride, to the oral cavity of a subject.
1.0.108. The preceding method wherein the basic amino acid is arginine.
1.0.109. A method of protecting against cavities comprising measuring the QLF or ECM value for a patient, and improving the QLF or ECM value for such patient to a greater extent than achievable using fluoride without arginine or using arginine without fluoride.
1.0.110. A method to reduce the size of existing early enamel lesions, comprising applying an effective amount of a basic amino acid, and an effective amount of fluoride, to the oral cavity of a subject.
1.0.111. The preceding method wherein the basic amino acid is arginine.
1.0.112. A method to improve systemic health, through the regular, application of an oral care product comprising applying an effective amount of a basic amino acid, and an effective amount of fluoride, to the oral cavity of a subject.
1.0.113. The preceding method wherein the basic amino acid is arginine.
1.0.114. A method as hereinbefore described which is additionally effective to
   a. reduce or inhibit formation of dental caries,
   b. reduce or inhibit demineralization and promote remineralization of the teeth,
   c. reduce hypersensitivity of the teeth,
   d. reduce or inhibit gingivitis,
   e. promote healing of sores or cuts in the mouth,
   f. reduce levels of acid producing bacteria,
   g. to increase relative levels of arginolytic bacteria,
   h. inhibit microbial biofilm formation in the oral cavity,
   i. raise and/or maintain plaque pH at levels of at least about pH 5.5 following sugar challenge,
   j. reduce plaque accumulation,
   k. treat, relieve or reduce dry mouth,
   l. whiten teeth,
   m. enhance systemic health, including cardiovascular health,
   n. reduce erosion of the teeth,
   o. immunize or protect the teeth against cariogenic bacteria, and/or
   p. clean the teeth and oral cavity.
   The invention further provides the use of a basic amino acid in the manufacture of a composition or combined preparation for use in a method as hereinbefore described, e.g., (i) the use of a basic amino acid, in free or salt form, and a fluoride, for the manufacture of a combined preparation for simultaneous, sequential or separate administration to the oral cavity of a subject for treating, reducing or inhibiting early enamel lesions; (ii) the use of a basic amino acid, in free or salt form, and a fluoride, for the manufacture of a combined preparation for simultaneous, sequential or separate administration to the oral cavity of a subject for reducing the size of early enamel lesions; and (iii) the use of a basic amino acid, in free or salt form, and a fluoride, for the manufacture of a combined preparation for simultaneous, sequential or separate administration to the oral cavity of a subject for improving systemic health, for example
1.0.115. Use of a basic amino acid, in free or salt form, and a fluoride, for the manufacture of a composition or combined preparation for simultaneous, sequential or separate administration to the oral cavity of a subject for treating, reducing or inhibiting early enamel lesions. wherein the early enamel lesions are detected by quantitative light-induced fluorescence (QLF) or electrical caries monitoring (ECM).
1.0.116. Use of a basic amino acid, in free or salt form, and a fluoride, for the manufacture of a composition or combined preparation for simultaneous, sequential or separate administration to the oral cavity of a subject to improve the QLF or ECM value, correlating to early enamel lesions.
1.0.117. The use of claim 40 wherein the basic amino acid is arginine.
1.0.118, Use of a basic amino acid, in free or salt form, and a fluoride, for the manufacture of a composition or combined preparation for simultaneous, sequential or separate administration to the oral cavity of a subject for protecting against cavities by measuring the QLF or ECM value for a patient, and improving the QLF or ECM value for such patient to a greater extent than achievable using fluoride without arginine or using arginine without fluoride.
1.0.119. The foregoing uses wherein the basic amino acid comprises arginine and is present in an amount of from 0.1 to 20 wt% of the total combined preparation weight.
1.0.120. The foregoing uses wherein the fluoride is present as a soluble fluoride salt in an amount of from 0.01 to 2 wt% of the total combined preparation weight.
1.0.121. The foregoing uses wherein the fluoride as a source of fluoride ions is present in an amount to provide 50 to 25,000 ppm by weight of fluoride ions in the total combined preparation weight.
1.0.122. The foregoing uses wherein the soluble fluoride salt or source of fluoride ions is selected from sodium fluoride, sodium monofluoropohosphate, and mixtures thereof
1.0.123. The foregoing uses wherein the composition or combined preparation further comprises a calcium salt in an amount of from 10 to 60wt% of the total combined preparation weight.
1.0.124. The preceding use wherein the calcium salt is a salt of an inorganic oxoacid.
1.0.125. The preceding use wherein the calcium salt comprises calcium phosphate.
1.0.126. Use of a basic amino acid, in free or salt form, and a fluoride, for the manufacture of a combined preparation for simultaneous, sequential or separate administration to the oral cavity of a subject for reducing the size of early enamel lesions.
1.0.127. The preceding use wherein the basic amino acid comprises arginine and is present in an amount of from 0.1 to 20 wt% of the total combined preparation weight, and the fluoride is present as a soluble fluoride salt in an amount of from 0.01 to 2 wt% of the total combined preparation weight or in an amount to provide 50 to 25,000 ppm by weight of fluoride ions in the total combined preparation weight.
1.0.128. Use of a basic amino acid, in free or salt form, and a fluoride, for the manufacture of a combined preparation for simultaneous, sequential or separate administration to the oral cavity of a subject for improving systemic health.
1.0.129. The preceding use wherein the basic amino acid comprises arginine and is present in an amount of from 0.1 to 20 wt% of the total combined preparation weight, and the fluoride is present as a soluble fluoride salt in an amount of from 0.01 to 2 wt% of the total combined preparation weight or in an amount to provide 50 to 25,000 ppm by weight of fluoride ions in the total combined preparation weight.

It has been found that the combined preparations and compositions as hereinbefore described can therefore be employed for treating, reducing or inhibiting early enamel lesions, and/or for improving the QLF or ECM value correlating to early enamel lesions and, when so employed, yield unexpected beneficial effects. It may therefore be seen by the skilled practitioner in the oral care art that a number of different yet surprising technical effects and advantages can result from the formulation, and use, of an oral care composition, for example a dentifrice, in accordance with one or more aspects of the invention, which are directed to the provision of different combinations of active components or ingredients, and preferably their respective amounts, within the composition.

Levels of active ingredients will vary based on the nature of the delivery system and the particular active. For example, the basic amino acid may be present at levels from, e.g., about 0.1 to about 20 wt %(expressed as weight of free base), e.g., about 0.1 to about 3 wt % for a mouthrinse, about 1 to about 10 wt % for a consumer toothpaste or about 7 to about 20 wt % for a professional or prescription treatment product. Fluoride may be present at levels of, e.g., about 25 to about 25,000 ppm, for example about 25 to about 250 ppm for a mouthrinse, about 750 to about 2,000 ppm for a consumer toothpaste, or about 2,000 to about 25,000 ppm for a professional or prescription treatment product. Levels of antibacterial will vary similarly, with levels used in toothpaste being e.g., about 5 to about 15 times greater than used in mouthrinse. For example, a triclosan mouthrinse may contain, e.g., about 0.03 wt % triclosan while a triclosan toothpaste may contain about 0.3 wt % triclosan.

### Basic Amino Acids

The basic amino acids which can be used in the compositions and methods of the invention include not only naturally occurring basic amino acids, such as arginine, lysine, and histidine, but also any basic amino acids having a carboxyl group and an amino group in the molecule, which are water-soluble and provide an aqueous solution with a pH of about 7 or greater.

Accordingly, basic amino acids include, but are not limited to, arginine, lysine, citruilene, ornithine, creatine, histidine, diaminobutanoic acid, diaminoproprionic acid, salts thereof or combinations thereof. In a particular embodiment, the basic amino acids are selected from arginine, citrullene, and ornithine.

In certain embodiments, the basic amino acid is arginine, for example, L-arginine, or a salt thereof.

In some embodiments the basic amino acid comprises at least one intermediate produced in the arginine deiminase system. The intermediates produced in the arginine deiminase system may be useful in an oral care composition to provide plaque neutralization for caries control and/or prevention. Arginine is a natural basic amino acid that may be found in the oral cavity. Arginine in the mouth may be utilized by certain dental plaque bacterial strains such as *S. sanguis, S. gordonii, S. parasanguis, S. rattus, S. milleri, S. anginosus, S. faecalis, A. naeslundii, A. odonolyticus, L. cellobiosus, L. brevis, L. fermentum, P. gingivalis,* and *T. demicola* for their survival. Such organisms may perish in an acidic environment that may be present at areas close to the tooth surface where acidogenic and aciduric cariogenic strains may use sugars to produce organic acids. Thus, these arginolytic strains may break down arginine to ammonia to provide alkalinity to survive and, in addition, buffer the plaque and make a hostile environment for the cariogenic systems.

Such arginolytic organisms may catabolize arginine by an internal cellular enzyme pathway system called the "arginine deiminase system" whereby intermediates in the pathway are formed. In this pathway, L-arginine may be broken down to L-citrulline and ammonia by arginine deiminase. L-citrulline may then be broken down by ornithane trancarbamylase in the presence of inorganic phosphate to L-ornithine and carbamyl phosphate. Carbamate kinase may then break down carbamyl phosphate to form another molecule of ammonia and carbon dioxide. and in the process also forms ATP (adenosine 5'-triphosphate). ATP may be used by the arginolytic bacteria as an energy source for growth. Accordingly, when utilized, the arginine deiminase system may yield two molecules of ammonia.

It has been found that, in some embodiments, the ammonia may help in neutralizing oral plaque pH to control and/or prevent dental caries.

The oral care composition of some embodiments of the present invention may include intermediates produced in the arginine deiminase system. Such intermediates may include citrulline, ornithine, and carbamyl phosphate. In some embodiments, the other care composition includes citrulline. In some embodiments, the oral care composition includes ornithine. In some embodiments, the oral care composition includes carbamyl phosphate. In other embodiments, the oral care composition includes any combination of citrulline, ornithine, carbamyl phosphate, and/or other intermediates produced by the arginine deiminase system.

The oral care composition may include the above described intermediates in an effective amount. In some embodiments, the oral care composition includes about 1 mmol/L to about 10 mmol/L intermediate. In other embodiments, the oral care composition includes about 3 mmol/L to about 7 mmol/L intermediate. In other embodiments, the oral care composition includes about 5 mmol/L intermediate.

The compositions of the invention are intended for topical use in the mouth and so salts for use in the present invention should be safe for such use, in the amounts and concentrations provided. Suitable salts include salts known in the art to be pharmaceutically acceptable salts are generally considered to be physiologically acceptable in the amounts and concentrations provided. Physiologically acceptable salts include those derived from pharmaceutically acceptable inorganic or organic acids or bases, for example acid addition salts formed by acids which form a physiological acceptable anion, e.g., hydrochloride or bromide salt, and base addition salts formed by bases which form a physiologically acceptable cation, for example those derived from alkali metals such as potassium and sodium or alkaline earth metals such as calcium and magnesium. Physiologically acceptable salts may be obtained using standard procedures known in the art, for example, by reacting a sufficiently basic compound such as an amine with a suitable acid affording a physiologically acceptable anion.

In various embodiments, the basic amino acid is present in an amount of about 0.5 wt. % to about 20 wt. % of the total composition weight, about 1 wt. % to about 10 wt. % of the total composition weight, for example about 1,5 wt. %, about 3.75 wt. %, about 5 wt. %. or about 7.5 wt. % of the total composition weight.

RDA: RDA is an abbreviation for radioactive dentin abrasion, a relative measure of abrasivity. Typically, extracted human or cow teeth are irradiated in a neutron flux, mounted in methylmethacrylate (bone glue), stripped of enamel, inserted into a brushing-machine, brushed by American Dental Association (ADA) standards (reference toothbrush, 150g pressure, 1500 strokes, 4-to-1 water-toothpaste slurry). The radioactivity of the rinsewater is then measured and recorded. For experimental control, the test is repeated with an ADA reference toothpaste made of calcium pyrophosphate, with this measurement given a value of 100 to calibrate the relative scale.

Fluoride Ion Source: The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al., incorporated herein by reference.

Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof.

In certain embodiments, the oral care composition of the invention may also contain a source of fluoride ions or fluorine-providing ingredient in amounts sufficient to supply about 25 ppm to about 25,000 ppm of fluoride ions, generally at least about 500 ppm, e.g., about 500 to about 2000 ppm, e.g., about 1000 to about 1600 ppm, e.g., about 1450 ppm. The appropriate level of fluoride will depend on the particular application. A mouthwash, for example, would typically have about 100 to about 250 ppm fluoride, A toothpaste for general consumer use would typically have about 1000 to about 1500 ppm, with pediatric toothpaste having somewhat less. A dentifrice or coating for professional application could have as much as about 5,000 or even about 25,000 ppm fluoride.

Fluoride ion sources may be added to the compositions of the invention at a level of about 0.01 wt. % to about 10 wt. % in one embodiment or about 0.03 wt. % to about 5 wt. %, and in another embodiment about 0.1 wt. % to about 1 wt. % by weight of the composition in another embodiment. Weights of fluoride salts to provide the appropriate level of fluoride ion will obviously vary based on the weight of the counter ion in the salt.

### Abrasives

The Compositions of the Invention may comprise a calcium phosphate abrasive, e.g., tricalcium phosphate (Ca₃(PO₄)₂), hydroxyapatite (Ca₁₀(PO₄)₆(OH)₂), or dicalcium phosphate dihydrate (CaHPO₄ • 2H₂O, also sometimes referred to herein as DiCal) or calcium pyrophosphate. Alternatively, calcium carbonate, and in particular precipitated calcium carbonate, may be employed as an abrasive.

The compositions may include one or more additional abrasives, for example silica abrasives such as precipitated silicas having a mean particle size of up to about 20 microns, such as Zeodent 115^{®} marketed by J. M. Huber. Other useful abrasives also include sodium metaphosphate, potassium metaphosphate, aluminum silicate, calcined alumina, bentonite or other siliceous materials, or combinations thereof.

The silica abrasive polishing materials useful herein, as well as the other abrasives, generally have an average particle size ranging between about 0.1 and about 30 microns, about between 5 and about 15 microns. The silica abrasives can be from precipitated silica or silica gels, such as the silica xerogels described in U.S. Pat. No. 3,538,230, to Pader et al. and U.S. Pat. No. 3,862,307, to Digiulio, both incorporated herein by reference. Particular silica xerogels are marketed under the trade name Syloid^{®} by the W. R. Grace & Co., Davison Chemical Division. The precipitated silica materials include those marketed by the J. M. Huber Corp. under the trade name Zeodent^{®}, including the silica carrying the designation Zeodent 115 and 119. These silica abrasives are described in U.S. Pat. No. 4,340,583, to Wason, incorporated herein by reference.

In certain embodiments, abrasive materials useful in the practice of the oral care compositions in accordance with the invention include silica gels and precipitated amorphous silica having an oil absorption value of less than about 100 cc/100 g silica and in the range of about 45 cc/100 g to about 70 cc/100 g silica. Oil absorption values are measured using the ASTA Rub-Out Method D281. In certain embodiments, the silicas are colloidal particles having an average particle size of about 3 microns to about 12 microns, and about 5 to about 10 microns.

In particular embodiments, the abrasive materials comprise a large fraction of very small particles, e.g., having a d50 < 5 microns, for example, small particle silica (SPS) having a d50 of about 3 to about 4 microns, for example Sorbosil AC43^{®} (Ineos). Such small particles are particularly useful in formulations targeted at reducing hypersensitivity. The small particle component may be present in combination with a second larger particle abrasive. In certain embodiments, for example, the formulation comprises about 3 to about 8% SPS and about 25 to about 45% of a conventional abrasive.

Low oil absorption silica abrasives particularly useful in the practice of the invention are marketed under the trade designation Sylodent XWA^{®} by Davison Chemical Division of W.R. Grace & Co., Baltimore, Md. 21203. Sylodent 650 XWA^{®}, a silica hydrogel composed of particles of colloidal silica having a water content of 29% by weight averaging about 7 to about 10 microns in diameter, and an oil absorption of less than about 70 cc/100 g of silica is an example of a low oil absorption silica abrasive useful in the practice of the present invention. The abrasive is present in the oral care composition of the present invention at a concentration of about 10 to about 60% by weight, in other embodiment about 20 to about 45% by weight, and in another embodiment about 30 to about 50% by weight.

In some embodiments the basic amino acid is incorporated into a dentifrice composition having a base formulation comprising calcium carbonate, and in particular precipitated calcium carbonate, as an abrasive. L-arginine and arginine salts such as arginine bicarbonate are themselves distinctly bitter in taste, and in aqueous solution can also impart a fishy taste. Consequently, it is expected that when L-arginine or arginine salts are incorporated into oral care products such as dentifrice formulations at effective concentrations to impart anticavity efficacy and sensitivity relief, typically in an amount of from 2 to 10wt % based on the total weight of the dentifrice formulation, the taste and mouth feel of the dentifrice formulations would be degraded as compared to the same formulation without the addition of L-arginine or arginine salts.

However, it has surprisingly been found in accordance with this aspect of the present invention that the addition of L-arginine or arginine salts to a base dentifrice formulation comprising calcium carbonate can provide a significant enhancement of taste and mouthfeel attributes to the dentifrice formulation and to an increase in the overall acceptance of the product to a consumer.

### Agents to increase the Amount of Foaming

The oral care compositions of the invention also may include an agent to increase the amount of foam that is produced when the oral cavity is brushed.

Illustrative examples of agents that increase the amount of foam include, but are not limited to polyoxyethylene and certain polymers including, but not limited to, alginate polymers.

The polyoxyethylene may increase the amount of foam and the thickness of the foam generated by the oral care carrier component of the present invention. Polyoxycthylene is also commonly known as polyethylene glycol ("PEG") or polyethylene oxide. The polyoxyethylenes suitable for this invention will have a molecular weight of about 200,000 to about 7,000,000. In one embodiment the molecular weight will be about 600,000 to about 2,000,000 and in another embodiment about 800,000 to about 1,000,000. Polyox^{®} is the trade name for the high molecular weight polyoxyethylene produced by Union Carbide.

The polyoxyethylene may be present in an amount of about 1% to about 90%, in one embodiment about 5% to about 50% and in another embodiment about 10% to about 20% by weight of the oral care carrier component of the oral care compositions of the present invention. The dosage of foaming agent in the oral care composition (i.e., a single dose) is about 0.01 to about 0.9 % by weight, about 0.05 to about 0.5% by weight, and in another embodiment about 0.1 to about 0.2 % by weight.

### Surfactants

The compositions useful in the invention may contain anionic surfactants, for example
i. water-soluble salts of higher fatty acid monoglyceride monosulfates, such as the sodium salt of the monosulfated monoglyceride of hydrogenated coconut oil fatty acids such as sodium N-methyl N-cocoyl taurate, sodium cocomo-glyceride sulfate,
ii. higher alkyl sulfates, such as sodium lauryl sulfate,
iii. higher alkyl-ether sulfates, e.g., of formula CH₃(CH₂)ₘCH₂(OCH₂CH₂)ₙOSO₃X, wherein m is 6-16, e.g., 10, n is 1-6, e.g., 2, 3 or 4, and X is Na or K. for example sodium laureth-2 sulfate (CH₃(CH₂)₁₀CH₂(OCH₂CH₂)₂OSO₃Na).
iv. higher alkyl aryl sulfonates such as sodium dodecyl benzene sulfonate (sodium lauryl benzene sulfonate)
v. higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate.

By "higher alkyl" is meant, e.g., C₆₋₃₀ alkyl. In particular embodiments, the anionic surfactant is selected from sodium lauryl sulfate and sodium ether lauryl sulfate.

The anionic surfactant may be present in an amount which is effective, e.g., > 0.01% by weight of the formulation, but not at a concentration which would be irritating to the oral tissue, e.g., < 10%, and optimal concentrations depend on the particular formulation and the particular surfactant. For example, concentrations used or a mouthwash are typically on the order of one tenth that used for a toothpaste. In one embodiment, the anionic surfactant is present in a toothpaste at from about 0.3% to about 4.5% by weight, e.g., about 1.5%.

The Compositions of the Invention may optionally contain mixtures of surfactants, comprising anionic surfactants and other surfactants which may be anionic, cationic, zwitterionic or nonionic. Generally, surfactants are those which are reasonably stable throughout a wide pH range. Surfactants are described more fully, for example, in U.S. Pat. No. 3,959,458, to Agricola et al.; U.S. Pat. No. 3,937,807, to Haefele; and U.S. Pat. No. 4,051,234, to Gieske et al., which are incorporated herein by reference.

In certain embodiments, the anionic surfactants useful herein include the water-soluble salts of alkyl sulfates having about 10 to about 18 carbon atoms in the alkyl radical and the water-soluble salts of sulfonated monoglycerides of fatty acids having about 10 to about 18 carbon atoms. Sodium lauryl sulfate, sodium lauroyl sarcosinate and sodium coconut monoglyceride sulfonates are examples of anionic surfactants of this type. Mixtures of anionic surfactants may also be utilized,

In another embodiment cationic surfactants useful in the present invention can be broadly defined as derivatives of aliphatic quaternary ammonium compounds having one long alkyl chain containing about 8 to about 18 carbon atoms such as lauryl trimethylammonium chloride, cetyl pyridinium chloride, cetyl trimethylammonium bromide, di-isobutylphenoxyethyldimethylbenzylammonium chloride, coconut alkyltrimethylammonium nitrite, cetyl pyridinium fluoride, and mixtures thereof.

Illustrative cationic surfactants are the quaternary ammonium fluorides described in U.S. Pat. No. 3,535,421. to Briner et al., herein incorporated by reference. Certain cationic surfactants can also act as germicides in the compositions.

Illustrative nonionic surfactants that can be used in the compositions of the invention can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

In certain embodiments, zwitterionic synthetic surfactants useful in the present invention can be broadly described as derivatives of aliphatic quaternary ammonium, phosphomium, and sulfonium compounds, in which the aliphatic radicals can be straight chain or branched, and wherein one of the aliphatic substituents contains about 8 to about 18 carbon atoms and one contains an anionic water-solubilizing group, e.g., carboxy, sulfonate, sulfate, phosphate or phosphonate. Illustrative examples of the surfactants suited for inclusion into the composition include, but are not limited to, sodium alkyl sulfate, sodium lauroyl sarcosinate, cocoamidopropyl betaine and polysorbate 20, and combinations thereof.

In a particular embodiment, the Composition used in the method of the Invention comprises sodium lauryl sulfate.

The surfactant or mixtures of compatible surfactants can be present in the compositions of the present invention in about 0.1 % to about 5.0%. in another embodiment about 0.3% to about 3.0% and in another embodiment about 0.5% to about 2.0% by weight of the total composition.

### Flavoring Agents

The oral care compositions of the invention may also include a flavoring agent. Flavoring agents which are used in the practice of the present invention include, but are not limited to, essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

The flavoring agent is incorporated in the oral composition at a concentration of about 0.1 to about 5% by weight and about 0.5 to about 1.5% by weight. The dosage of flavoring agent in the individual oral care composition dosage (i.e., a single dose) is about 0.001 to 0.05% by weight and in another embodiment about 0.005 to about 0.015 % by weight.

### Chelating agents

The oral care compositions of the invention also may optionally include one or more chelating agents able to complex calcium found in the cell walls of the bacteria. Binding of this calcium weakens the bacterial cell wall and augments bacterial lysis.

Another group of agents suitable for use as chelating agents in the present invention are the soluble pyrophosphates. The pyrophosphate salts used in the present compositions can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The salts are useful in both their hydrated and unhydrated forms. An effective amount of pyrophosphate salt useful in the present composition is generally enough to provide at least about 1.0 wt. % pyrophosphate ions, about 1.5 wt. % to about 6 wt. %, about 3.5 wt. % to about 6 wt. % of such ions.

### Polymers

The oral care compositions of the invention also optionally include one or more polymers, such as polyethylene glycols, polyvinylmethyl ether maleic acid copolymers, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts.

Particularly when noncationic antibacterial agents or antibacterial agents, e.g., triclosan, are included in any of the dentifrice components, there is also preferably included from about 0.05 to about 5% of an agent which enhances the delivery and retention of the agents to, and retention thereof on oral surfaces. Such agents useful in the present invention are disclosed in U.S. Pat. Nos. 5,188,821 and 5, 192,531; and include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W.) of about 30,000 to about 1,000,000, most preferably about 30,000 to about 800,000. These copolymers are available for example as Gantrez. e.g., AN 139 (M.W. 500,000), AN 119 (M.W. 250,000) and preferably S-97 Pharmaceutical Grade (M.W. 700,000) available from ISP Technologies, Inc., Bound Brook, N.J. 08805. The enhancing agents when present are present in amounts ranging from about 0.05 to about 3% by weight.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1 103, M.W. 10,000 and EMA Grade 61, and I:I copolymers of acrylic acid with methyl or hydroxyethyl methacrylate, methyl or ethyl acrylate, isobutyl vinyl ether or N-vinyl-2-pyrrolidone.

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid, incorporated herein by reference.
Another useful class of polymeric agents includes polyamino acids, particularly those containing proportions of anionic surface-active amino acids such as aspartic acid, glutamic acid and phosphoserine, as disclosed in U.S. Pat. No. 4,866,161 Sikes et al., incorporated herein by reference.

In preparing oral care compositions, it is sometimes necessary to add some thickening material to provide a desirable consistency or to stabilize or enhance the performance of the formulation. In certain embodiments, the thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate or finely divided silica can be used as component of the thickening composition to further improve the composition's texture. In certain embodiments, thickening agents in an amount of about 0.5% to about 5.0% by weight of the total composition are used.

### Enzymes

The oral care compositions of the invention may also optionally include one or more enzymes. Useful enzymes include any of the available proteases, glucanohydrolases, endoglycosidases, amylases, mutanases, lipases and mucinases or compatible mixtures thereof. In certain embodiments, the enzyme is a protease, dextranase, endoglycosidase and mutanase. In another embodiment, the enzyme is papain, endoglycosidase or a mixture of dextranase and mutanase. Additional enzymes suitable for use in the present invention are disclosed in U.S. Pat. No. 5,000,939 to Dring et al., U.S. Pat. No. 4,992,420; U.S. Pat. No. 4,355,022; U.S. Pat. No. 4,154,815; U.S. Pat. No. 4,058,595; U.S. Pat. No. 3,991,177; and U.S. Pat. No. 3,696,191 all incorporated herein by reference. An enzyme of a mixture of several compatible enzymes in the current invention constitutes about 0.002% to about 2.0% in one embodiment or about 0.05% to about 1.5% in another embodiment or in yet another embodiment about 0.1% to about 0.5%.

### Water

Water may also be present in the oral compositions of the invention. Water, employed in the preparation of commercial oral compositions should be deionized and free of organic impurities. Water commonly makes up the balance of the compositions and includes about 10% to about 90%, about 20% to about 60% or about 10% to about 30% by weight of the oral compositions. This amount of water includes the free water which is added plus that amount which is introduced with other materials such as with sorbitol or any components of the invention.

### Humectants

Within certain embodiments of the oral compositions, it is also desirable to incorporate a humectant to prevent the composition from hardening upon exposure to air. Certain humectants can also impart desirable sweetness or flavor to dentifrice compositions. The humectant, on a pure humectant basis, generally includes about 15% to about 70% in one embodiment or about 30% to about 65% in another embodiment by weight of the dentifrice composition.

Suitable humectants include edible polyhydric alcohols such as glycerine, sorbitol, xylitol, propylene glycol as well as other polyols and mixtures of these humectants. Mixtures of glycerine and sorbitol may be used in certain embodiments as the humectant component of the toothpaste compositions herein.

In addition to the above described components, the embodiments of this invention can contain a variety of optional dentifrice ingredients some of which are described below. Optional ingredients include, for example, but are not limited to, adhesives, sudsing agents, flavoring agents, sweetening agents, additional antiplaque agents, abrasives, and coloring agents. These and other optional components are further described in U.S. Pat. No. 5,004,597, to Majeti; U.S. Pat. No. 3,959,458 to Agricola et al. and U.S. Pat. No. 3,937,807, to Haefele, all being incorporated herein by reference.

### Methods of Manufacture

The compositions of the present invention can be made using methods which are common in the oral product area.

In one illustrative embodiment, the oral care composition is made by neutralizing or partially neutralizing arginine in a gel phase with an acid, e.g., phosphoric acid, hydrochloric acid or carbonic acid, and mixing to form Premix 1.

Actives such as, for example, vitamins, CPC, fluoride, abrasives, and any other desired active ingredients are added to Premix 1 and mixed to form Premix 2.

Where the final product is a toothpaste, a toothpaste base, for example, dicalcium phosphate or silica, is added to Premix 2 and mixed. The final slurry is formed into an oral care product.

The compositions and methods according to the invention can be incorporated into oral compositions for the care of the mouth and teeth such as toothpastes, transparent pastes, gels, mouth rinses, sprays and chewing gum.

The invention provides method to reduce early lesions of the enamel (as measured by QLF or ECM) relative to a composition lacking effective amounts of fluorine and/or arginine.

These methods additionally reduce harmful bacteria in the oral cavity, for example methods to reduce or inhibit gingivitis, reduce levels of acid producing bacteria, to increase relative levels of arginolytic bacteria, inhibit microbial biofilm formation in the oral cavity, raise and/or maintain plaque pH at levels of at least about pH 5.5 following sugar challenge, reduce plaque accumulation, and/or clean the teeth and oral cavity.

Finally, by increasing the pH in the mouth and discouraging pathogenic bacteria, the methods of the invention are useful to promote healing of sores or cuts in the mouth.

Enhancing oral health also provides benefits in systemic health, as the oral tissues can be gateways for systemic infections. Good oral health is associated with systemic health, including cardiovascular health. The compositions and methods of the invention provide particular benefits because basic amino acids, especially arginine, are sources of nitrogen which supply NO synthesis pathways and thus enhance microcirculation in the oral tissues. Providing a less acidic oral environment is also helpful in reducing gastric distress and creates an environment less favorable to Heliobacter, which is associated with gastric ulcers. Arginine in particular is required for high expression of specific immune cell receptors, for example T-cell receptors, so that arginine can enhance an effective immune response. The compositions and methods of the invention are thus useful to enhance systemic health, including cardiovascular health.

As used throughout, ranges are used as shorthand for describing each and every value that is within the range. Any value within the range can be selected as the terminus of the range. In addition, all references cited herein are hereby incorporated by reference in their entireties. In the event of a conflict in a definition in the present disclosure and that of a cited reference, the present disclosure controls. It is understood that when formulations are described, they may be described in terms of their ingredients, as is common in the art, notwithstanding that these ingredients may react with one another in the actual formulation as it is made, stored and used, and such products are intended to be covered by the formulations described.

The following examples further describe and demonstrate illustrative embodiments within the scope of the present invention. The examples are given solely for illustration and are not to be construed as limitations of this invention as many variations are possible without departing from the spirit and scope thereof. Various modifications of the invention in addition to those shown and described herein should be apparent to those skilled in the art and are intended to fall within the appended claims.

### EXAMPLES

### Example 1 - Efficacy in Remineralization

The neutralized dicalcium phosphate / arginine phosphate / fluoride formulation is tested against dicalcium phosphate / fluoride formulations without arginine in a clinical study of demineralization / remineraiization.

The intra-oral demin-remin study is a short term study used to assess anticaries technologies. In this model enamel specimens experience conditions of demineralization and remineralization in the mouth. Demineralizing conditions are created by dipping the specimens in s sugar solution. The cariogenic bacteria form acids and cause the pH to drop. In this model, blocks of bovine specimens that have been polished flat to a mirror finish are prepared. A micro hardness tester is used to measure the hardness of the enamel specimen at baseline (M1). The micro hardness tester uses a diamond tipped probe to create an indent in the enamel specimen with a known and constant load. The length of the indent is inversely related to the enamel hardness. Enamel hardness is directly correlated with the mineral content. The specimens are covered with a Dacron mesh and then mounted in a retainer. The specimens are worn 24 hours per day for 5 days. During the 5 day period, the panelists dip their retainer in a sucrose solution 4 times per day. This treatment causes the pH fluctuations. The panelists brush their teeth two times per day with the assigned dentifrice while the retainer is in the mouth. After 5 days, the specimens are removes from the retainer and a micro hardness measurement is conducted (M2). The plaque can be further analyzed for plaque ecology or plaque metabolism measurements. Because of the highly cariogenic condition created by dipping the specimens sugar 4 times per day, most treatments tend to experience a net loss in mineral after the 5 day treatment, hence, the name "demin-remin model". The best treatment loses the least amount of mineral. There are circumstances, however, where a net increase in hardness is achieved with a particularly effective treatment.

The statistical analysis is a two factor analysis using the subject and treatment as factors. The results can be expressed as a %change in hardness (M2-M1)/M1 x100 or a net change in hardness M2-M1. If a percent change is used as the measured response, a two factor ANOVA is conducted. If a net change in hardness is used, a two factor ANCOVA is conducted using M1 as the covariate. Differences are considered significant if a 95% confidence level is achieved. Typically a 250 ppm fluoride (or nonfluoride) and a dentifrice with a standard level of fluoride are included as negative and positive controls and are used to validate the model. The fluoride level in the positive control is most commonly 1000, 1100, or 1450 ppm fluoride. The control chosen is dependent on the fluoride level in the test dentifrice. The model is considered validated if positive control is shown to be significantly better than the negative control. Once the model is validated, the test product is compared to the negative control. It should be noted that the panelist effect is normally very significant; therefore, it is not expected that the same numerical result for an identical treatment will be obtained using a different study population.

| Formulation | % mineral change |
|---|---|
| Dical + 250 MFP | -12.7 |
| Dical + 1450 MFP | -1.87 |
| Dical + 1.5% neutralized arginine + 1450 MFP | +8.27 |

### (Example 3)

Notes:
Dical = Dicalcium phosphate dihydrate
MFP = sodium monofluorophosphate, units in ppm fluoride

The neutralized dicalcium phosphate / arginine phosphate / fluoride formulation is the only formulation to show an actual increase mineralization in this clinical study.

### Example 2 - Mouthrinse formulations

Mouthwash formulations useful in the invention are prepared using the following ingredients:
Arginine Rinse with Fluoride, SLS,
PMV/MA, and Triclosan

| RAW MATERIAL | WEIGHT % |
|---|---|
| Deionized Water | q.s. |
| Glycerin | 15.000 |
| Sodium methyl cocoyl taurate | 0.250 |
| 95% Ethanol | 6.000 |
| Sodium lauryl sulfate | 0.200 |
| Allantoin | 0.110 |
| Sodium benzoate | 0.100 |
| Sodium salicylate | 0.100 |
| Sodium fluoride | 0.050 |
| Sodium Saccharin | 0.005 |
| Triclosan | 0.030 |
| Phosphoric acid 85% | 0.120 |
| L-Arginine | 0.300 |
| Flavor | 0.100 |
| Colorants | 0.001 |
| PVM/MA | 0.250 |
| TOTAL | 100.000 |
| pH | 7.0 |

### Example 3 - Dentifrice formulation comprising precipitated calcium carbonate (PCC)

A panel of consumer testers trained in testing the sensory attributes of dentifrice formulations is subjected to different dentifrice formulations which are used under double-blind consumer testing conditions replicating consumer use of dentifrice formulations.

The panel is asked to use the dentifrice formulations conventionally and then to rate various sensory characteristics. For a base dentifrice formulation comprising precipitated calcium carbonate (PCC), the known formulation acted as a placebo control, and corresponding formulations additionally comprising 1, 2, 3 or 5 wt% arginine bicarbonate are also tested. Surprisingly, it is found that the arginine bicarbonate-containing PCC formulations exhibited increases in consumer acceptance for flavor intensity, cooling and ease to foam attributes, and moreover the formulation additionally comprising 2 wt% arginine bicarbonate exhibits increases in overall liking, overall liking of taste, taste while brushing and taste after brushing. In addition, the formulations additionally comprising arginine bicarbonate are perceived as significantly better than the placebo control in all image attributes, including perceived efficacy, mouth/teeth feeling of clean, product suitability, taste and overall product quality.

The Example shows that the addition of a basic amino acid such as arginine, in particular as bicarbonate, can surprisingly enhance the sensory characteristics of dentifrice formulations, most particularly having a base formulation of precipitated calcium carbonate (PCC), when used in an oral care composition of the invention.

### Example 4 - Basic amino acids other than arginine

An overnight culture of *S*. *sanguis* is grown at 37°C in trypticase soy broth (Becton Dickinson, Sparks, MD). The culture is centrifuged at 5,000 rpm for 5 minutes at 1 milliliter at a time into preweighed tubes in order to accumulate approximately 5 milligrams of wet pellet weight. The pellet is then resuspended into 20 millimolar potassium phosphate buffer (JT Baker, Phillipsburg, NJ), pH 4.0, to simulate a stressed environment for the bacterial cell where ammonia would be produced for survival. The final concentration is 5 milligram per milliliter. To this final concentration, a 5 millimolar final concentration of L-arginine, L-citrulline, or L-ornithine is added along with a 0.1% final concentration of sucrose (VWR, West Chester, PA). This mixture is then incubated at 37°C in a shaking water bath for 30 minutes before ammonia production is determined.

In order to analyze for ammonia, an Ammonia Assay kit is used from Diagnostic Chemicals Limited (Oxford, CT). The intended use of this specific kit is for the *in vitro* quantification of ammonia in plasma, but the procedure is modified in order to determine and quantify the ammonia production in plaque and/or bacteria.

The table below shows the ammonia production values from 6 separate trials using *S. sanguis* at pH 4.0 as described above. The results confirm that the intermediates produced by the arginine deiminase system can be used to produce ammonia for cell survival.

| | **L-Arginine** | **L-Citrulline** | **L-Ornithine** |
|---|---|---|---|
| **Trial #** | **Ammonia (ppm)** | **Ammonia (ppm)** | **Ammonia (ppm)** |
| 1 | 0.509 | 0.185 | 0.185 |
| 2 | 0.866 | 0.346 | 0.260 |
| 3 | 2.20 | 0.332 | 0.047 |
| 4 | 1.62 | 0.194 | 0.0 |
| 5 | 0.5 | 0.226 | 0.181 |
| 6 | 0.679 | 0.951 | 0.135 |
| Mean | 1.06 | 0.951 | 0.134 |

The Example shows that basic amino acids other than arginine are effective to produce ammonia within the oral cavity, and thus to increase plaque pH thereby reducing early enamel lesions when used in a oral care composition of the invention,.

The following description pages 38 to 40 contain preferred embodiments. Accordingly, the term "claim" as used therein refers to such a "preferred embodiment".

## Claims

**1.** A method of treating or reducing early enamel caries comprising applying an effective amount of an oral composition comprising a basic amino acid, in free or salt form, and an effective amount of fluoride, to the oral cavity.

**2.** The method of claim 1 wherein the basic amino acid is arginine.

**3.** The method of claim 1 or claim 2 wherein the early enamel caries are detected by quantitative light-induced fluorescence (QLF) or electrical caries monitoring (ECM).

**4.** A method to improve the QLF or ECM value, correlating to early enamel lesions, comprising applying an effective amount of a basic amino acid a basic amino acid, arginine, in free or salt form, and an effective amount of fluoride, to the oral cavity of a subject.

**5.** The method of claim 4 wherein the basic amino acid is arginine.

**6.** A method of protecting against cavities comprising measuring the QLF or ECM value for a patient, and improving the QLF or ECM value for such patient to a greater extent than achievable using fluoride without arginine or using arginine without fluoride.

**7.** A method to reduce the size of existing early enamel lesions, comprising applying an effective amount of a basic amino acid, and an effective amount of fluoride, to the oral cavity of a subject.

**8.** A method of claim 7 wherein the basic amino acid is arginine.

**9.** A method to improve systemic health, through the regular, application of an oral care product comprising applying an effective amount of a basic amino acid, and an effective amount of fluoride, to the oral cavity of a subject.

**10.** A method of claim 9 wherein the basic amino acid is arginine.

**11.** A method of any of the preceding claims which is additionally effective to
q, reduce or inhibit formation of dental caries,
r. reduce or inhibit demineralization and promote remineralization of the teeth,
s. reduce hypersensitivity of the teeth,
t. reduce or inhibit gingivitis,
u. promote healing of sores or cuts in the mouth,
v. reduce levels of acid producing bacteria,
w. to increase relative levels of arginolytic bacteria,
x. inhibit microbial biofilm formation in the oral cavity,
y. raise and/or maintain plaque pH at levels of at least about pH 5.5 following sugar challenge,
z. reduce plaque accumulation,
aa. treat, relieve or reduce dry mouth,
bb. whiten teeth,
cc. enhance systemic health, including cardiovascular health,
dd. reduce erosion of the teeth,
ee. immunize or protect the teeth against cariogenic bacteria, and/or
ff. clean the teeth and oral cavity.

**12.** The use of a basic amino acid in the manufacture of a composition or combined preparation for use in a method according to any of claims 1-12.

**13.** Use of a basic amino acid, in free or salt form, and a fluoride, for the manufacture of a composition or combined preparation for simultaneous, sequential or separate administration to the oral cavity of a subject for treating, reducing or inhibiting early enamel lesions, wherein the early enamel lesions are detected by quantitative light-induced fluorescence (QLF) or electrical caries monitoring (ECM).

**14.** Use of a basic amino acid, in free or salt form, and a fluoride, for the manufacture of a composition or combined preparation for simultaneous, sequential or separate administration to the oral cavity of a subject to improve the QLF or ECM value, correlating to early enamel lesions.

**15.** The use of claim 13 or 14 wherein the basic amino acid is arginine.

**16.** Use of a basic amino acid, in free or salt form, and a fluoride, for the manufacture of a composition or combined preparation for simultaneous, sequential or separate administration to the oral cavity of a subject for protecting against cavities by measuring the QLF or ECM value for a patient, and improving the QLF or ECM value for such patient to a greater extent than achievable using fluoride without arginine or using arginine without fluoride.

**17.** A combined preparation comprising a basic amino acid, in free or salt form, and a fluoride, for simultaneous, sequential or separate administration to the oral cavity of a subject for treating, reducing or inhibiting early enamel lesions.

**18.** A combined preparation comprising a basic amino acid, in free or salt form, and a fluoride, for simultaneous, sequential or separate administration to the oral cavity of a subject for improving the QLF or ECM value correlating to early enamel lesions.

**1.** A composition for use in reducing or inhibiting demineralization and promoting remineralization of the teeth, comprising a basic amino acid, in free or salt form, and an effective amount of fluoride, wherein the basic amino acid is arginine, and wherein the basic amino acid is present in an amount corresponding to 0.1 to 20% of the total composition weight, the weight of the basic amino acid being calculated as free base form.

**2.** The composition of claim 1, wherein the basic amino acid is present in an amount corresponding to 1 to 10% of the total composition weight.

**3.** The composition of claim 2, wherein the basic amino acid is present in an amount of about 1.5 wt. % of the total composition weight.

**4.** The composition of any of claims 1 to 3, wherein the fluoride is selected from stannous fluoride, sodium fluoride, potassium fluoride, sodium monofluorophosphate. sodium fluorosilicate, ammonium fluorosilicate, amine fluoride, ammonium fluoride, titanium fluoride, hexafluorosulfate, and combinations thereof.

**5.** The composition of any of claims 1 to 4, wherein the fluoride provides fluoride ion in an amount of from 50 to 10,000 ppm.

**6.** The composition of any of claims 1 to 5, wherein the fluoride provides fluoride ion in an amount of from about 1450 ppm.

**7.** The composition of any of claims 1 to 6, wherein the dentifrice comprises an abrasive selected from precipitated calcium carbonate, dicalcium phosphate dihydrate and a combination thereof.

**8.** The composition of claim 7, wherein the abrasive is in an amount of 15 wt.% to 70 wt.% of the total composition weight.

**9.** The composition of any of claims 1 to 8, wherein the dentifrice composition comprises a potassium ion source.

**10.** The composition of claim 9, wherein the potassium ion source is selected from potassium nitrate and potassium chloride.

**11.** A composition comprising arginine phosphate, dicalcium phosphate dihydrate and sodium monofluorophosphate.

**12.** The composition of claim 11 for use in reducing or inhibiting demineralization and promoting remineralization of the teeth.

**13.** The composition of claim 11 or 12, wherein the arginine is present in an amount corresponding to 1 to 10% of the total composition weight, the weight of the arginine being calculated as free base form.

**14.** The composition of any of claims 11 to 13, wherein the fluoride provides fluoride ion in an amount of from 50 to 10,000 ppm.
